# EUROPEAN PATENT SPECIFICATION

(11) **EP 3 136 400 B1**
(45) Date of publication and mention of the grant of the patent: **15.11.2017**
(21) Application number: 16184209.1
(22) Date of filing: 15.08.2016
(51) Int. Cl.: G21F 7/00, A61N 5/10

(54) **NEUTRON CAPTURE THERAPY SYSTEM**
THERAPIESYSTEM MIT NEUTRONENERFASSUNG
SYSTÈME DE THÉRAPIE PAR CAPTURE DE NEUTRONS

(30) Priority: 25.08.2015 JP 2015166004
(43) Date of publication of application: 01.03.2017
(73) Proprietor: SUMITOMO HEAVY INDUSTRIES, LTD., Tokyo 141-6025 (JP); KYOTO UNIVERSITY, Kyoto 606-8501 (JP)
(72) Inventor: MITSUMOTO, Toshinori, Nishitokyo-shi, Tokyo 188-8585 (JP); SUZUKI, Minoru, Kyoto-shi, Kyoto 606-8501 (JP); TANAKA, Hiroki, Kyoto-shi, Kyoto 606-8501 (JP)
(74) Representative: Louis Pöhlau Lohrentz

(56) References cited:
- WO-A1-2005/120640
- WO-A1-2007/009786
- WO-A1-2014/132501

## Description

### Field of the Invention

The invention is as defined in the appended claims. The present invention relates to a neutron capture therapy system in which an irradiation target is irradiated with a neutron beam.

### Description of the Related Art

Patent document WO2014132501 discloses a neutron capture therapy system arrangement including shielding walls; Japanese Unexamined Patent Application Publication No. 2011-185784 discloses a neutron capture therapy apparatus which is utilized in the neutron capture therapy (NCT). In NCT treatment using this apparatus, first, a medicine is given to a patient. The medicine contains a substance which reacts to a neutron such as ¹⁰B and has properties of being selectively incorporated into a cancer cell to be treated. When the patient who has taken the medicine is irradiated with a neutron beam, a reactant incorporated into the cancer cell of the patient and the neutron beam react to each other, and thus, heavy charged particles are generated. When the heavy charged particles are scattered, only the cancer cell is destroyed.

In the above-described therapeutics, from the viewpoint of improving therapeutic efficacy, it is desired that a medicine is supplied to a patient by using an apparatus such as an infusion pump apparatus not only before irradiation of a neutron beam but also during the irradiation of a neutron beam. However, inside an irradiation chamber having a neutron beam of a high radiation dose, a device including electronic components may cause an erroneous operation. In such a case, electronic components inside the infusion pump apparatus cause an erroneous operation, and it is difficult to supply an appropriate quantity of the medicine to the patient. Thus, it is no longer possible to expect improvement of therapeutic efficacy through supplying of the medicine during irradiation.

### SUMMARY OF THE INVENTION

In consideration of the aforementioned circumstances, the present invention aims to provide a neutron capture therapy system which can improve therapeutic efficacy.

According to an aspect of the present invention, there is provided a neutron capture therapy system in which an irradiation target is irradiated with a neutron beam. The neutron capture therapy system includes an irradiation chamber which is surrounded by a shielding wall blocking the neutron beam from the inside to the outside of the chamber and in which the irradiation target is arranged inside the chamber and is irradiated with the neutron beam, a neutron beam irradiation unit that irradiates the inside of the irradiation chamber with the neutron beam, a shielding door that closes a gate provided in the shielding wall, a medicine supply device that supplies a medicine to the irradiation target, and a medicine supply tube through which the medicine is introduced from the medicine supply device to the irradiation target arranged inside the irradiation chamber. The medicine supply device has a control unit which controls a supply quantity of the medicine and is arranged outside the irradiation chamber.

In the neutron capture therapy system, the neutron beam irradiation unit irradiates an irradiation target with a neutron beam. In this case, the irradiation target is arranged in the irradiation chamber, and a medicine is applied to the irradiation target from the medicine supply device. Since the medicine supply device has the control unit, a desired quantity of the medicine can be supplied to the irradiation target. Moreover, the medicine supply device is arranged outside the irradiation chamber which is surrounded by the shielding wall. Accordingly, the radiation dose irradiating the medicine supply device is reduced compared to when the medicine supply device is arranged inside the irradiation chamber, and an erroneous operation can be prevented from occurring. Therefore, a desired quantity of the medicine is reliably supplied during irradiation, and thus, therapeutic efficacy can be improved.

In addition, in a state where the shielding door blocks the gate, a groove-shaped tube arrangement portion in which the medicine supply tube is drawn out from the inside to the outside of the irradiation chamber is formed on an end surface of the shielding door or in a region covered by the end surface. According to such a tube arrangement portion, the medicine supply tube can be easily arranged through an opening portion of the groove. In addition, when the shielding door is open, the state of the medicine supply tube arranged in the tube arrangement portion can be visually recognized.

In addition, a hole-shaped tube arrangement portion through which the medicine supply tube is drawn out from the inside to the outside of the irradiation chamber is formed in the shielding wall or the shielding door. According to such a tube arrangement portion, the tube arrangement portion is not arranged in the gate through which the irradiation target passes. Therefore, no irregularity is present on the surface of the gate, and thus, the irradiation target can be moved safely.

In addition, the tube arrangement portion has a first part which extends in a first direction, and a secondpart which extends in a second direction intersecting the first direction and is connected to the first part. According to such a tube arrangement portion, in a connecting portion between the first part and the second part in the tube arrangement portion, extending directions thereof in the tube arrangement portion are different from each other. Consequently, in a case where a neutron beam transmitted straight invades the tube arrangement portion, the neutron beam collides with a wall in the connecting portion between the first part and the second part. Therefore, it is possible to prevent the neutron beam from being radiated from the inside to the outside of the irradiation chamber.

According to the neutron capture therapy system of the present invention, therapeutic efficacy can be improved.

### BRIEF DESCRIPTION OF THE DRAWINGS

FIG. 1 is a plan view schematically illustrating a neutron capture therapy system.
FIG. 2 is an enlarged plan view of an irradiation chamber, a communication chamber, and a preparation chamber illustrated in FIG. 1.
FIG. 3 is a perspective view illustrating a tube arrangement portion, according to a first embodiment.
FIG. 4 is a perspective view illustrating a tube arrangement portion, according to a second embodiment.
FIG. 5 is a plan view illustrating a tube arrangement portion, according to a third embodiment.
FIG. 6 is a side view illustrating a tube arrangement portion, according to a fourth embodiment.
FIGS. 7A to 7D are views illustrating tube arrangement portions, according to modification examples.
FIGS. 8A to 8C are views illustrating tube arrangement portions, according to modification examples.

### DETAILED DESCRIPTION OF THE INVENTION

### <First Embodiment>

Hereinafter, embodiments of the present invention will be described in detail, with reference to the drawings. In the description below, the same reference symbols will be applied to the same or corresponding elements, and the description will not be repeated. In each of the drawings, an XYZ coordinate system is set, and X, Y, and Z will be applied in the description of a positional relationship of each of the configuration elements. Here, an X-axis is an emitting direction of a neutron beam N emitted from a neutron beam irradiation unit 8. A Z-axis is a direction perpendicular to a floor surface. Inaddition, a Y-axis is a direction orthogonal to both the emitting direction of the neutron beam N (X-axis direction) and the direction perpendicular to the floor surface (Z-axis direction).

A neutron capture therapy system is an apparatus which performs cancer treatment by applying boron neutron capture therapy (BNCT). In the neutron capture therapy, the cancer treatment is performed by irradiating a patient (irradiation target) who has taken boron (¹⁰B), with a neutron beam.

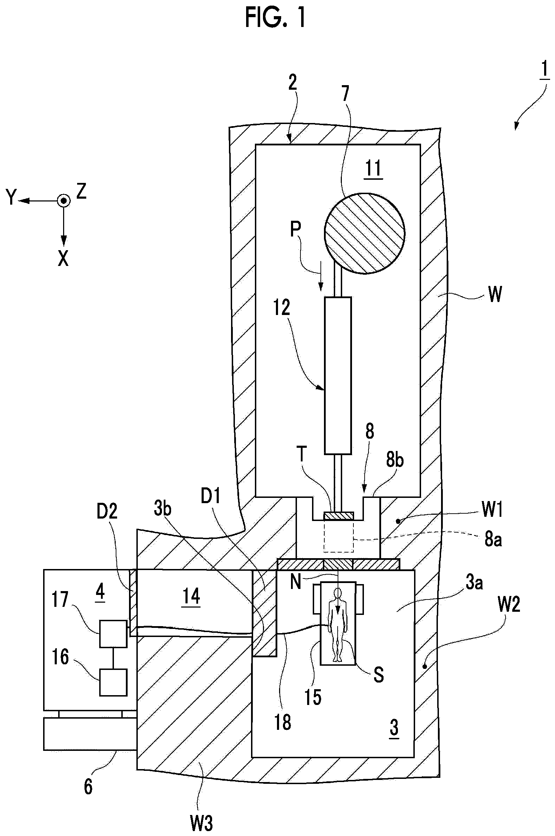

As illustrated in FIG. 1, a neutron capture therapy system 1 includes a neutron beam generation unit 2 which generates the therapeutic neutron beam N so as to perform irradiation, an irradiation chamber 3 in which a patient S is irradiated with the neutron beam N, a preparation chamber 4 in which irradiation is prepared, and a management chamber 6 in which a work process is managed.

The neutron beam generation unit 2 is configured to generate the neutron beam N inside the irradiation chamber 3 so as to be able to irradiate the patient S with the neutron beam N. The neutron beam generation unit 2 includes an accelerator 7 such as a cyclotron, the neutron beam irradiation unit 8 which generates the neutron beam N from a charged particle beam P, and a beam transportation path 12 through which the charged particle beam P is transported to the neutron beam irradiation unit 8. The accelerator 7 and the beam transportation path 12 are arranged inside a charged particle beam generation chamber 11. The charged particle beam generation chamber 11 is an enclosed space covered by a concrete shielding wall W.

The accelerator 7 accelerates charged particles which are protons, and produces a charged particle beam P which is a proton beam, thereby emitting the charged particle beam P. For example, the accelerator 7 is capable of generating a charged particle beam P having the beam radius of 40 mm, and 60kW (= 30 MeV X 2 mA).

The beam transportation path 12 emits the charged particle beam P to the neutron beam irradiation unit 8. One end side of the beam transportation path 12 is connected to the accelerator 7, and the other end side thereof is connected to the neutron beam irradiation unit 8. As necessary, the beam transportation path 12 may be provided with beam control devices such as a beam adjustment unit, a current monitor, and a charged particle scanning unit. The beam adjustment unit controls the advancing direction and the beam diameter of the charged particle beam P. The current monitor measures current values (that is, an electric charge, and an irradiated radiation dose rate) of the charged particle beam P in real time. The charged particle beam scanning unit performs scanning with the charged particle beam P and controls an irradiation position of the charged particle beam P with respect to a target T.

The neutron beam irradiation unit 8 includes the target T for generating the neutron beam N, a speed-reducing member 8a for decelerating the neutron beam N, and a shielding body 8b. The speed-reducing member 8a and the shielding body 8b are configured to form a moderator.

Here, the neutron beam N generated in the neutron beam irradiation unit 8 includes a fast neutron beam, an epithermal neutron beam, a thermal neutron beam, and a gamma ray. Mainly, the thermal neutron beam thereamong has a nuclear reaction with boron incorporated into a tumor inside the body of the patient S exhibits an effective therapeutic effect. A portion of the epithermal neutron beam included in the neutron beam N is also decelerated inside the body of the patient S and becomes the thermal neutron beam exhibiting the therapeutic effect. The thermal neutron beam is a neutron beam having energy equal to or less than 0.5 eV.

The target T is irradiated with the charged particle beam P and generates the neutron beam N. For example, the target T is formed from beryllium (Be) and has a disc shape with the diameter of 160 mm.

The speed-reducing member 8a decelerates the neutron beam N emitted from the target T. The neutron beam N which has been decelerated by the speed-reducing member 8a and has been reduced in energy to a predetermined level is also called as a therapeutic neutron beam. For example, the speed-reducing member 8a has a stacked structure formed of multiple materials different from each other. The materials of the speed-reducing member 8a are suitably selected based on various conditions such as energy of the charged particle beam P.

For example, in a case where an output from the accelerator 7 is a proton beam of 30 MeV and a beryllium target is applied as the target T, lead, iron, aluminum, or calcium fluoride can be adopted as the materials of the speed-reducing member 8a. In addition, in a case where an output from the accelerator 7 is a proton beam of 11 MeV and a beryllium target is applied as the target T, heavy water (D20) or lead fluoride can be adopted as the materials of the speed-reducing member 8a. In addition, in a case where an output from the accelerator 7 is a proton beam of 2.8 MeV and a lithium target is applied as the target T, Fluental (brand name; a mixture of aluminum, aluminum fluoride, and lithium fluoride) can be adopted as the materials of the speed-reducing member 8a. In addition, in a case where an output from the accelerator 7 is a proton beam of 50 MeV and a tungsten target is applied as the target T, iron or Fluental can be adopted as the materials of the speed-reducing member 8a.

The shielding body 8b performs shielding such that radiation (for example, the neutron beam N or a gamma ray caused in response to generation of the neutron beam N) is not released to the outside . At least a portion thereof is embedded in a shielding wall W1 separating the charged particle beam generation chamber 11 and the irradiation chamber 3 from each other.

In the neutron capture therapy system 1, the irradiation chamber 3 and the preparation chamber 4 are connected to each other via a communication chamber 14.

The irradiation chamber 3 is a room in which the patient S is arranged inside the chamber in order to irradiate the patient S with the neutron beam N. The irradiation chamber 3 is arranged on the extended line in a direction in which the beam transportation path 12 extends. As an example, the size of the irradiation chamber 3 measures the width 3.5 m X the depth 5 m X the height 3 m. The irradiation chamber 3 is configured to have a shielded space 3a surrounded by a shielding wall W2, a gate 3b through which the patient S goes in and out, and a shielding door D1 by which the gate 3b is open and closed.

The shielding wall W2 forms the shielded space 3a. In the shielded space 3a, radiation is prevented from invading from the outside to the inside of the irradiation chamber 3, and radiation is prevented from being released from the inside to the outside of the irradiation chamber 3. That is, the shielding wall W2 blocks the neutron beam N from being radiated from the inside to the outside of the irradiation chamber 3. The shielding wall W2 is formed integrally with the shielding wall W which defines the charged particle beam generation chamber 11. In addition, the shielding wall W2 is a concrete wall having the thickness equal to or greater than 2 m. The shielding wall W1 separating the charged particle beam generation chamber 11 and the irradiation chamber 3 from each other is provided between the charged particle beam generation chamber 11 and the irradiation chamber 3. The shielding wall W1 is a portion of the shielding wall W.

The gate 3b is provided in a portion of the shielding wall W2. The shielding door D1 is arranged in the gate 3b. The shielding door D1 prevents radiation in the shielded space 3a from being radiated to the communication chamber 14. The shielding door D1 is formed of a radiation shielding member such as lead. The shielding door D1 moves on a rail provided inside the irradiation chamber 3 due to drive force applied by a motor or the like. Since the shielding door D1 is heavy, a high torque motor, a speed reducer, or the like is used as a mechanism for driving the shielding door D1.

The shielding door D1 may have a function of issuing notification regarding going in and out of a worker with respect to the irradiation chamber 3. For example, in a state where the treatment platform 15 is arranged inside the irradiation chamber 3, evacuation of the worker from the irradiation chamber 3 may be checked when the shielding door D1 is closed.

The preparation chamber 4 is a room in which preparation work required in irradiating the patient S with the neutron beam N is executed. For example, the preparation work includes constraint on the patient S with respect to the treatment platform 15, and positioning of a collimator and the patient S. The preparation chamber 4 is arranged so as to be separated from the irradiation chamber 3 in a Y-axis direction. A shielding wall W3 separating the preparation chamber 4 and the irradiation chamber 3 from each other is provided between the preparation chamber 4 and the irradiation chamber 3. For example, the shielding wall W3 has a thickness of 3.2 m. That is, the preparation chamber 4 and the irradiation chamber 3 are separated from each other by 3.2 m along the Y-axis direction.

The preparation chamber 4 maybe a shielded space surrounded by the shielding wall W, similar to the irradiation chamber 3, and may be a non-shielded space not surrounded by the shielding wall W.

The communication chamber 14 allowing the preparation chamber 4 and the irradiation chamber 3 to communicate with each other is provided in the shielding wall W3. The communication chamber 14 is a room through which the patient S constrained onto the treatment platform 15 is moved between the preparation chamber 4 and the irradiation chamber 3. As an example, the size of the communication chamber 14 measures the width 1.5 m X the depth 3.2 m X the height 2.0 m. A door D2 is arranged between the preparation chamber 4 and the communication chamber 14.

The neutron capture therapy system 1 includes the management chamber 6. The management chamber 6 is a room in which the overall process executed by using the neutron capture therapy system 1 is managed. A supervisor enters the management chamber 6 and manages the overall process by using a control device for operating monitoring equipment and the neutron beam generation unit 2 arranged inside the management chamber 6. For example, the supervisor enters the management chamber 6 and visually checks the state of preparation work in the preparation chamber 4 from the inside of the management chamber 6. In addition, the supervisor operates the control device. For example, the supervisor controls the start and the stop of irradiation of the neutron beam N by operating the control device.

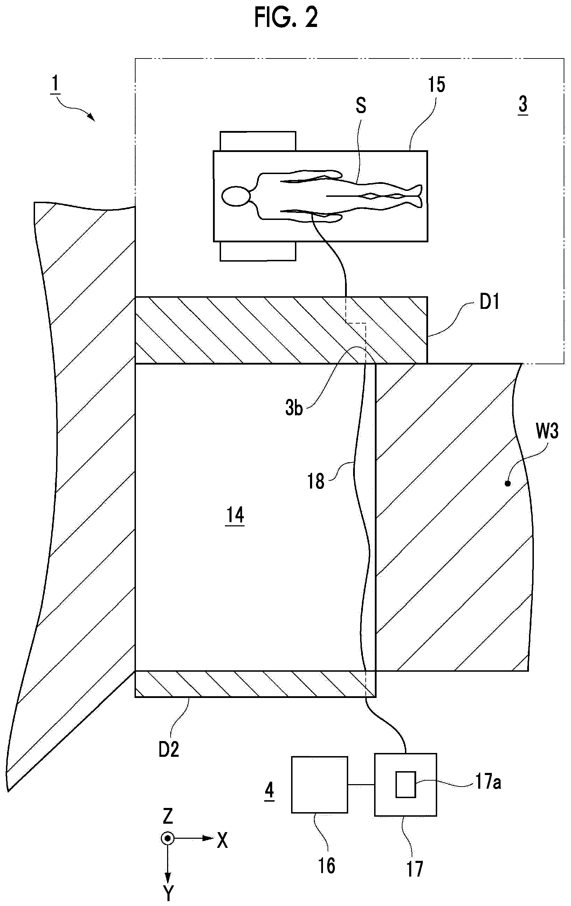

As illustrated in FIG. 2, the neutron capture therapy system 1 includes a medicine supply pump (medicine supply device) 17 for supplying a medicine held in an infusion solution bag 16 to the patient S, and a medicine supply tube 18 through which the medicine is introduced from the medicine supply pump 17 to the patient S. Due to the medicine supply pump 17 and the medicine supply tube 18 included therein, a medicine can be supplied to the patient S arranged in the irradiation chamber 3, during irradiation of the neutron beam N.

For example, in a case where one therapy is performed for one patient S, a medicine of 500 mL is given. In the quantity of the medicine, 80% (400 mL) of the medicine is given before irradiation of the neutron beam N, and 20% (100 mL) thereof is given during the irradiation. In addition, the medicine starts being given to the patient S from the medicine supply pump 17 two hours before the irradiation, and stops being given upon completion of the irradiation. Consequently, the medicine supply pump 17 has a function of controlling the supply quantity per unit time, and the control function is realized by a control unit 17a such as a microcomputer which is mounted inside the medicine supply pump 17 and is configured to have semiconductor components. In addition, the medicine supply pump 17 may have a function of issuing a warning when various types of abnormal states are detected, in addition to the function of controlling the supply quantity.

The medicine supply tube 18 is a tube having an outer diameter ranging from 2.0 mm to 4.0 mm. One end of the medicine supply tube 18 is connected to the medicine supply pump 17, and an injection needle such as a winged needle and an indwelling needle is connected to the other end thereof. The medicine is supplied to the patient S via the injection needle. Here, the medicine supply pump 17 is arranged in the preparation chamber 4, and the patient S is arranged in the irradiation chamber 3. In other words, the medicine supply pump 17 is arranged outside the irradiation chamber 3. Therefore, the medicine supply tube 18 connecting the medicine supply pump 17 and the patient S extends from the preparation chamber 4 to the irradiation chamber 3 via the communication chamber 14.

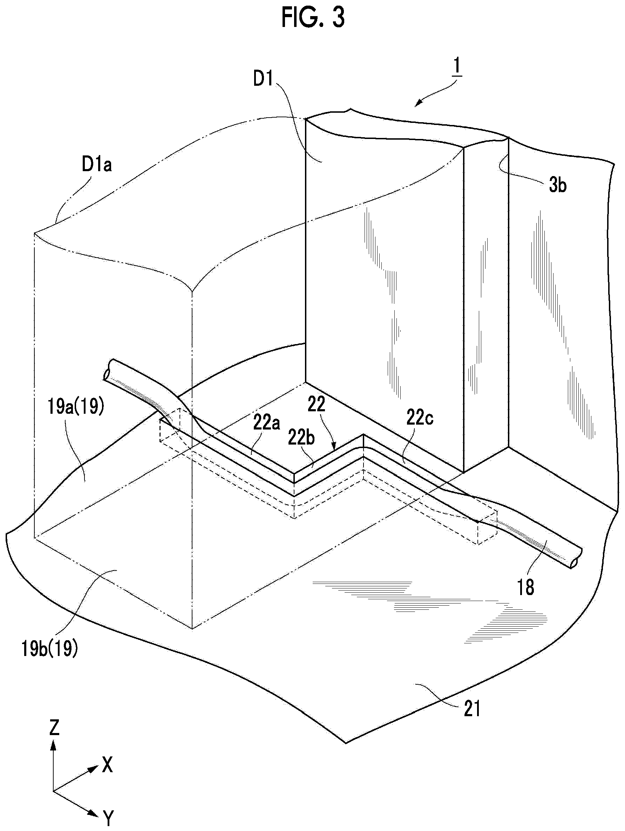

As illustrated in FIG. 3, the neutron capture therapy system 1 has a maze-shaped tube arrangement groove (tube arrangement portion) 22 which is formed on a floor 19 of the irradiation chamber 3 and a floor 21 of the communication chamber 14. The tube arrangement groove 22 allows the medicine supply tube 18 to be drawn out from the irradiation chamber 3 to the communication chamber 14 while preventing the medicine supply tube 18 from being squashed by the shielding door D1 such that a flow path of the medicine is not blocked, in a state where the shielding door D1 is closed (shielding door D1a indicated by the imaginary line in FIG. 3).

The tube arrangement groove 22 has a groove shape with an open top rectangular cross-section. The tube arrangement groove 22 has a groove width substantially equal to or slightly smaller than the outer diameter of the medicine supply tube 18. According to such a groove width, without blocking the flow path of the medicine, the medicine supply tube 18 can be held by being interposed between the groove side surfaces. In addition, the tube arrangement groove 22 has a groove depth substantially equal to the outer diameter of the medicine supply tube 18 or greater than the outer diameter of the medicine supply tube 18. According to such a groove depth, the medicine supply tube 18 does not protrude from the tube arrangement groove 22, and thus, the flow path of the medicine can be prevented from being blocked due to the squashed medicine supply tube 18, when the shielding door D1 is in an enclosed state.

In addition, the tube arrangement groove 22 has a flat crank-like shape in a planar view, and has a first part 22a, a second part 22b, and a third part 22c. When the shielding door D1 is in an enclosed state, the first part 22a extends in a thickness direction of the shielding door D1, that is, the Y-axis direction (first direction) from a floor region 19a which is not covered by the shielding door D1 to a floor region 19b which is covered by an end surface of the shielding door D1. The second part 22b leads to the first part 22a and extends in a direction orthogonal to the thickness direction of the shielding door D1, that is, the X-axis direction (second direction). That is, the second part 22b is connected to the first part 22a such that an extending direction of the tube arrangement groove 22 is bent. The third part 22c leads to the second part 22b and extends in the thickness direction of the shielding door D1 from the floor region 19b to the floor 21 of the communication chamber 14 which is not covered by the shielding door D1, when the shielding door D1 is in an enclosed state. That is, the third part 22c is connected to the second part 22b such that the extending direction of the tube arrangement groove 22 is bent.

In the neutron capture therapy system 1, the patient S is irradiated with the neutron beam N from the neutron beam irradiation unit 8. In this case, the patient S is arranged in the irradiation chamber 3, and a medicine is supplied from the medicine supply pump 17. Since the medicine supply pump 17 has the control unit 17a, a desired quantity of the medicine can be supplied to the patient S. Moreover, the medicine supply pump 17 arranged outside the irradiation chamber 3 surrounded by the shielding wall W2. Here, when a semiconductor component such as a microcomputer is irradiated with radiation, phenomena such as a total dose effect, a displacement cascade damage effect, and a single event effect may occur and can lead to an erroneous operation of the control unit 17a. In contrast, since the medicine supply pump 17 is arranged outside the irradiation chamber 3 surrounded by the shielding wall W2, the radiation dose irradiating the medicine supply pump 17 is reduced compared to when being arranged inside the irradiation chamber 3. Therefore, the control unit 17a can be prevented from causing an erroneous operation due to the irradiated radiation, and thus, it is possible to improve therapeutic efficacy by reliably supplying a desired quantity of the medicine during irradiation.

Moreover, in the neutron capture therapy system 1, compared to other radiation therapy systems, the inside of the irradiation chamber 3 is likely to be in a radio-activation state. However, in the neutron capture therapy system 1, the medicine supply pump 17 is arranged outside the irradiation chamber 3 which is likely to be in the radio-activation state, and thus, the radiation dose irradiating the medicine supply pump 17 is reduced compared to when being arranged inside the irradiation chamber 3. Therefore, in the neutron capture therapy system 1, it is possible to improve therapeutic efficacy by reliably supplying a desired quantity of the medicine during irradiation.

In addition, the tube arrangement groove 22 through which the medicine supply tube 18 is drawn out from the inside to the outside of the irradiation chamber 3 is formed between the shielding door D1 and the shielding wall W2 in a state where the gate 3b is blocked. According to such a tube arrangement groove 22, the medicine supply tube 18 can be easily arranged through the opening portion of the groove. In addition, when the shielding door D1 is open, the medicine supply tube 18 arranged in the tube arrangement groove 22 can be visually recognized.

In addition, the maze-shaped tube arrangement groove 22 has the first part 22a which extends in the Y-axis direction, and the second part 22b which extends in the X-axis direction and is connected to the first part 22a. According to such a tube arrangement groove 22, the extending directions of the tube arrangement groove 22 are different from each other in the connecting portion between the first part 22a and the second part 22b in the tube arrangement groove 22. Consequently, in a case where the neutron beam N which has passed through the first part 22a invades the tube arrangement groove 22, the neutron beam N collides with the wall and is drastically attenuated in the connecting portion between the first part 22a and the second part 22b. Therefore, it is possible to prevent the neutron beam N from being radiated from the inside to the outside of the irradiation chamber 3.

In addition, a person can enter the preparation chamber 4 even during irradiation of the neutron beam N. Accordingly, when the medicine supply pump 17 is arranged in the preparation chamber 4, it is possible to monitor the state of the medicine supply pump 17 and to operate the medicine supply pump 17 during the irradiation of the neutron beam N. Therefore, the supply quantity of the medicine supplied from the medicine supply pump 17 during irradiation of the neutron beam N can be maintained in an optimal state, and thus, the therapeutic efficacy can be further enhanced.

### <Second Embodiment>

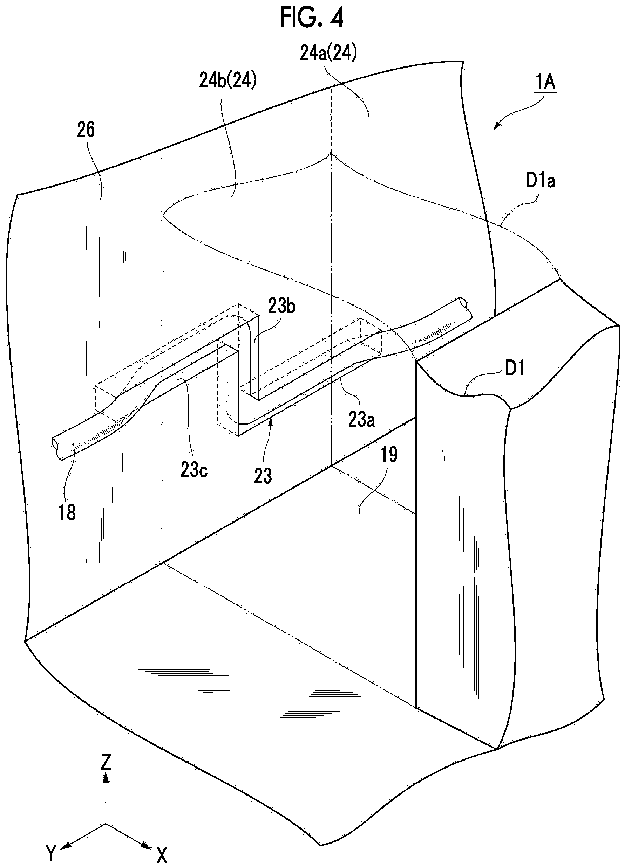

A neutron capture therapy system 1A according to a second embodiment will be described. As illustrated in FIG. 4, the neutron capture therapy system 1A is different from the neutron capture therapy system 1 in the point that a tube arrangement groove (tube arrangement portion) 23 is formed in a side wall 24 of the irradiation chamber 3 and a side wall 26 of the communication chamber 14. Other configurations are similar to those of the neutron capture therapy system 1. Hereinafter, the tube arrangement groove 23 will be described in detail.

The tube arrangement groove 23 has a groove width and a groove depth similar to those of the tube arrangement groove 22 in the first embodiment. The tube arrangement groove 23 has a crank-like shape in a planar view, and has a first part 23a on the irradiation chamber 3 side, a second part 23b, and a third part 23c on the communication chamber 14 side. The first part 23a extends along the thickness direction of the shielding door D1, that is, the Y-axis direction (first direction) from a region 24a to a region 24b in the side wall 24 of the irradiation chamber 3. Here, when the shielding door D1 is in an enclosed state (shielding door D1a indicated by the two-dot chained line in FIG. 4), the region 24a is a region which is not covered by the shielding door D1 in the side wall 24 of the irradiation chamber 3. In addition, when the shielding door D1 is in an enclosed state, the region 24b is a region covered by the end surface of the shielding door D1. The second part 23b leads to the first part 23a and extends along the direction orthogonal to the thickness direction of the shielding door D1, that is, the Z-axis direction (second direction) intersecting the floor 19. The third part 23c leads to the second part 23b and extends along the thickness direction of the shielding door D1 from the region 24b to the side wall 26 of the communication chamber 14. Here, the side wall 26 of the communication chamber 14 is a region which is not covered by the shielding door D1 when the shielding door D1 is in an enclosed state.

According to the tube arrangement groove 23 which is formed in the side wall 24 of the irradiation chamber 3 and the side wall 26 of the communication chamber 14 as described above, no irregularity is caused on the floor 19 of the irradiation chamber 3 and the floor 21 of the communication chamber 14, and thus, the patient S can be moved safely.

### <Third Embodiment>

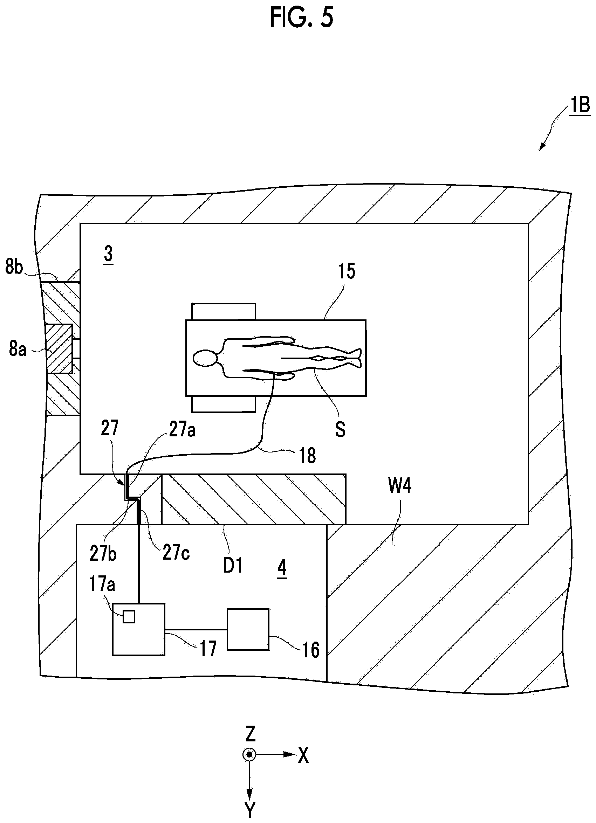

A neutron capture therapy system 1B according to a third embodiment will be described. As illustrated in FIG. 5, the neutron capture therapy system 1B is different from the neutron capture therapy system 1 in the points that the preparation chamber 4 is adjacent to the irradiation chamber 3 without arranging communication chamber 14, and a tube arrangement hole (tube arrangement portion) 27 is a through hole provided in a shielding wall W4 separating the irradiation chamber 3 and the preparation chamber 4 from each other. Other configurations are similar to those of the neutron capture therapy system 1. Hereinafter, the tube arrangement hole 27 will be described in detail.

The tube arrangement hole 27 has a hole diameter equal to or slightly greater than the outer diameter of the medicine supply tube 18. According to such a hole diameter, it is possible to easily execute work of inserting the medicine supply tube 18 from the inside to the outside of the irradiation chamber 3.

The tube arrangement hole 27 has a crank-like shape in a planar view, and has a first part 27a, a second part 27b, and a third part 27c. The first part 23a extends in the thickness direction of the shielding door D1, that is, the Y-axis direction (first direction) . The second part 23b leads to the first part 23a and extends in the direction orthogonal to the thickness direction of the shielding door D1, that is, the X-axis direction (second direction). The second direction may be a direction intersecting the floor 19 (that is, the Z-axis direction). The third part 27c leads to the second part 27b and extends in the thickness direction of the shielding door D1.

According to the tube arrangement hole 27 which is formed in the shielding wall W4 as described above, no irregularity is caused on the floor 19 of the irradiation chamber 3 and the floor of the preparation chamber 4, and thus, the patient S can be moved safely. In addition, since the tube arrangement hole 27 can be provided at an arbitrary place in the shielding wall W4, the degree of freedom can be enhanced when the tube arrangement hole 27 is arranged.

### <Fourth Embodiment>

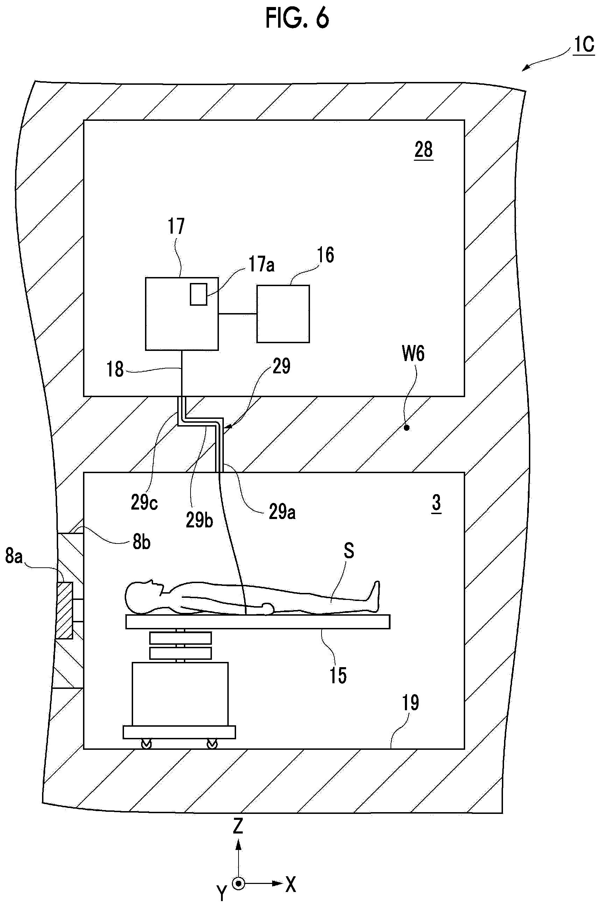

A neutron capture therapy system 1C according to a fourth embodiment will be described. As illustrated in FIG. 6, the neutron capture therapy system 1C is different from the neutron capture therapy system 1 in the points that a shielding chamber 28 surrounded by a shielding wall W6 is included above the irradiation chamber 3, and a tube arrangement hole (tube arrangement portion) 2 9 is a through hole provided in the shielding wall W6 separating the irradiation chamber 3 and the shielding chamber 28 from each other. Other configurations are similar to those of the neutron capture therapy system 1. Hereinafter, the tube arrangement hole 29 will be described in detail.

The tube arrangement hole 29 has a hole diameter equal to or slightly greater than the outer diameter of the medicine supply tube 18. According to such a hole diameter, it is possible to easily execute work of inserting the medicine supply tube 18 from the inside to the outside of the irradiation chamber 3.

The tube arrangement hole 29 has a crank-like shape in a side view, and has a first part 29a, a second part 29b, and a third part 29c. The first part 29a extends along a direction orthogonal to the floor 19, that is, the Z-axis direction (first direction). The second part 29b leads to the first part 29a and extends along a direction parallel to the floor 19, that is, the X-axis direction (second direction). The second direction may be the Y-axis direction. The third part 29c leads to the second part 29b and extends along the direction orthogonal to the floor 19.

According to the tube arrangement hole 29 which is formed in the side wall as described above, no irregularity is caused on the floor 19 of the irradiation chamber 3, and thus, the patient S can be moved safely.

The present invention is not limited to the above-described embodiments, and various types of changes can be made as described below without departing from the concept of the present invention.

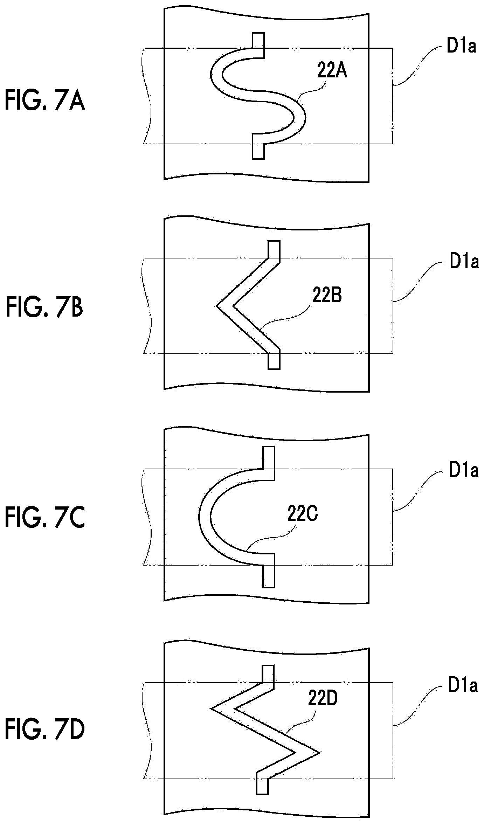

Without being limited to the crank-like shape, the shape thereof is acceptable as long as the tube arrangement groove 22 has a first part and a second part which extends in a direction different from the extending direction of the first part. For example, As illustrated in FIGS. 7(a) to 7(d), an S-shaped tube arrangement groove 22A in a planar view, a V-shaped tube arrangement groove 22B, an arc-shaped tube arrangement groove 22C, or a saw blade-shaped tube arrangement groove 22D may be adopted.

In addition, the first direction and the second direction are acceptable as long as the extending directions thereof are different from each other. The first direction is not limited to the thickness direction of the shielding door D1, and the second direction is not limited to the direction orthogonal to the thickness direction of the shielding door D1. For example, as illustrated in FIG. 7B, the first direction may be a direction which tilts with respect to the thickness direction of the shielding door D1, and the second direction may be a direction which intersects the first direction and tilts with respect to the thickness direction of the shielding door D1.

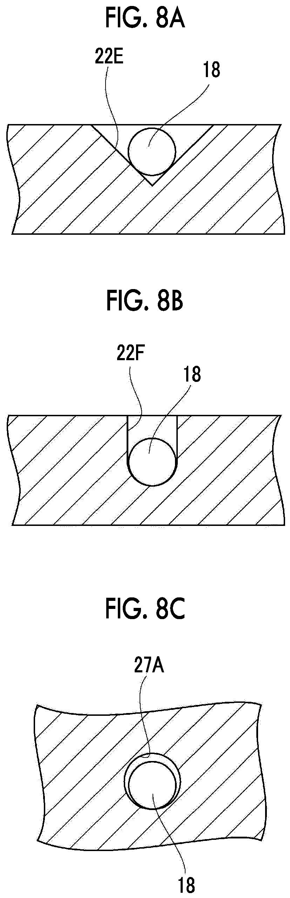

In addition, the cross-sectional shape of the tube arrangement groove 22 is not limited to the rectangular shape. As illustrated in FIGS. 8 (a) and 8(b), a tube arrangement groove 22E having a V-shaped cross section or a tube arrangement groove 22F having a U-shaped cross section may be adopted. In addition, the cross-sectional shape of the tube arrangement hole 27 is not limited to the rectangular shape. As illustrated in FIG. 8C, a tube arrangement hole 27A having a circular cross section may be adopted. The embodiments aspects and examples which do not fall within the scope of the claims are provided for illustrative purpose only and do not form part of the present invention. The invention is defined in the claims as follows.

### Brief Description of the Reference Symbols

1, 1A, 1B, 1C... NEUTRON CAPTURE THERAPY SYSTEM; 2... NEUTRON BEAM GENERATION UNIT; 3... IRRADIATION CHAMBER; 3b... GATE; 4... PREPARATION CHAMBER; 6... MANAGEMENT CHAMBER; 8... NEUTRON BEAM IRRADIATION UNIT; 12... BEAM TRANSPORTATION PATH; 14... COMMUNICATION CHAMBER; 17... MEDICINE SUPPLY PUMP (MEDICINE SUPPLY DEVICE) ; 17a... CONTROL UNIT; 18... MEDICINE SUPPLY TUBE; 22, 22A, 22B, 22C, 22D, 22E, 22F, 23... TUBE ARRANGEMENT GROOVE (TUBE ARRANGEMENT PORTION) ; 27, 2 7A, 29... TUBE ARRANGEMENT HOLE (TUBE ARRANGEMENT PORTION); D1... SHIELDING DOOR; N... NEUTRON BEAM; P... CHARGED PARTICLE BEAM; S... PATIENT; T... TARGET; and W, W1, W2, W3, W4, W6... SHIELDING WALL.

## Claims

1. A neutron capture therapy system (1) in which an irradiation target is irradiated with a neutron beam, the system comprising:
an irradiation chamber (3) which is surrounded by a shielding wall (W) blocking the neutron beam from being radiated from the inside to the outside of the chamber and in which the irradiation target is arranged inside the chamber and is irradiated with the neutron beam;
a neutron beam irradiation unit (8) that irradiates the inside of the irradiation chamber with the neutron beam;
a shielding door (D1) that closes a gate (3b) provided in the shielding wall (W); a medicine supply device (17) that supplies a medicine to the irradiation target; and
a medicine supply tube (18) through which the medicine is introduced from the medicine supply device to the irradiation target arranged inside the irradiation chamber (3) wherein the medicine supply device (17) has a control unit (17A). which controls a supply quantity of the medicine and is arranged outside the irradiation chamber (3).

2. The neutron capture therapy system (1) according to Claim 1,
wherein in a state where the shielding door blocks the gate, a groove-shaped tube arrangement portion (22) in which the medicine supply tube is drawn out from the inside to the outside of the irradiation chamber is formed on an end surface of the shielding door or in a region covered by the end surface.

3. The neutron capture therapy system (1) according to Claim 1,
wherein a hole-shaped tube arrangement portion (29) through which the medicine supply tube is drawn out from the inside to the outside of the irradiation chamber is formed in the shielding wall or the shielding door.

4. The neutron capture therapy system (1) according to Claim 2 or 3,
wherein the tube arrangement portion has a first part which extends in a first direction, and a second part which extends in a second direction intersecting the first direction and is connected to the first part.

## Patentansprüche

1. Neutroneneinfangtherapiesystem (1), in dem ein Bestrahlungstarget mit einem Neutronenstrahl bestrahlt wird, wobei das System Folgendes umfasst:
eine Bestrahlungskammer (3), die von einer Abschirmungswand (W), die den Neutronenstrahl so blockiert, dass er nicht von dem Innenraum zu dem Außenraum der Kammer strahlt, umgeben ist und in der das Bestrahlungstarget innerhalb der Kammer angeordnet ist und mit dem Neutronenstrahl bestrahlt wird;
eine Neutronenstrahlbestrahlungseinheit (8), die den Innenraum der Bestrahlungskammer mit dem Neutronenstrahl bestrahlt;
eine Abschirmungstür (D1), die ein Tor (3b), das in der Abschirmungswand (W) vorgesehen ist, schließt;
eine Arzneimittel-Zuführvorrichtung (17), die dem Bestrahlungstarget ein Arzneimittel zuführt; und
ein Arzneimittel-Zuführrohr (18), durch das das Arzneimittel von der Arzneimittel-Zuführvorrichtung zu dem Bestrahlungstarget, das innerhalb der Bestrahlungskammer (3) angeordnet ist, eingeleitet wird,
wobei die Arzneimittel-Zuführvorrichtung (17) eine Steuereinheit (17A), die eine Zuführmenge des Arzneimittels steuert und die außerhalb der Bestrahlungskammer (3) angeordnet ist, besitzt.

2. Neutroneneinfangtherapiesystem (1) nach Anspruch 1,
wobei in einem Zustand, in dem die Abschirmungstür das Tor blockiert, ein rillenförmiger Rohranordnungsabschnitt (22), in dem das Arzneimittel-Zuführrohr von dem Innenraum zu dem Außenraum der Bestrahlungskammer hinausgeführt wird, auf einer Endoberfläche der Abschirmungstür oder in einer Region, die durch die Endoberfläche bedeckt wird, gebildet ist.

3. Neutroneneinfangtherapiesystem (1) nach Anspruch 1,
wobei ein lochförmiger Rohranordnungsabschnitt (29), durch den das Arzneimittelzuführrohr von dem Innenraum zu dem Außenraum der Bestrahlungskammer hinausgeführt wird, in der Abschirmungswand oder der Abschirmungstür gebildet ist.

4. Neutroneneinfangtherapiesystem (1) nach Anspruch 2 oder 3,
wobei der Rohranordnungsabschnitt einen ersten Teil, der sich in einer ersten Richtung erstreckt, und einen zweiten Teil, der sich in einer zweiten Richtung, die die erste Richtung schneidet, erstreckt und mit dem ersten Teil verbunden ist, besitzt.

## Revendications

1. Système de thérapie par capture de neutrons (1) dans lequel une cible d'irradiation est irradiée avec un faisceau de neutrons, le système comprenant :
une chambre d'irradiation (3) qui est entourée par une paroi de blindage (W) empêchant le faisceau de neutrons d'être irradié de l'intérieur vers l'extérieur de la chambre et dans lequel la cible d'irradiation est agencée à l'intérieur de la chambre et est irradiée à l'aide du faisceau de neutrons ;
une unité d'irradiation de faisceau de neutrons (8) qui irradie l'intérieur de la chambre d'irradiation avec le faisceau de neutrons ;
une porte de blindage (D1) qui ferme une grille (3b) placée dans la paroi de blindage (W) ;
un dispositif de fourniture de médicament (17) qui fournit un médicament à la cible d'irradiation ; et
un tube de fourniture de médicament (18) à travers lequel le médicament est introduit du dispositif de fourniture de médicament vers la cible d'irradiation agencée à l'intérieur de la chambre d'irradiation (3)
dans lequel le dispositif de fourniture de médicament (17) possède une unité de commande (17a) qui commande une quantité de fourniture du médicament et est agencée à l'extérieur de la chambre d'irradiation (3).

2. Système de thérapie par capture de neutrons (1) selon la revendication 1,
dans lequel dans un état où la porte de blindage bloque la grille, une portion d'agencement de tube en forme de gorge (22) dans laquelle le tube de fourniture de médicament est retiré de l'intérieur vers l'extérieur de la chambre d'irradiation est formée sur une surface d'extrémité de la porte de blindage ou dans une région couverte par la surface d'extrémité.

3. Système de thérapie par capture de neutrons (1) selon la revendication 1,
dans lequel une portion d'agencement de tube en forme d'orifice (29) à travers laquelle le tube de fourniture de médicament est retiré de l'intérieur vers l'extérieur de la chambre d'irradiation est formée dans la paroi de blindage ou la porte de blindage.

4. Système de thérapie par capture de neutrons (1) selon la revendication 2 ou 3,
dans lequel la portion d'agencement de tube a une première partie qui s'étend dans une première direction, et une seconde partie qui s'étend dans une seconde direction croisant la première direction et est reliée à la première partie.
